# EUROPEAN PATENT SPECIFICATION

(11) **EP 4 140 010 B1**
(45) Date of publication and mention of the grant of the patent: **20.03.2024**
(21) Application number: 21717892.0
(22) Date of filing: 13.04.2021
(51) Int. Cl.: H01M 10/42, A24F 40/90, H01M 10/48, H02J 7/00

(54) **ELECTRICAL SYSTEM FOR AN AEROSOL GENERATING DEVICE**
ELEKTRISCHES SYSTEM FÜR EINE AEROSOLERZEUGUNGSVORRICHTUNG
SYSTÈME ÉLECTRIQUE POUR UN DISPOSITIF DE GÉNÉRATION D'AÉROSOL

(30) Priority: 22.04.2020 EP 20170908
(43) Date of publication of application: 01.03.2023
(73) Proprietor: JT International SA, 1202 Geneva (CH)
(72) Inventor: ZOMINY, Claude, 74350 Copponex (FR)
(74) Representative: Gill Jennings & Every LLP
(86) International application number: PCT/EP2021/059564
(87) International publication number: WO 2021/213848

(56) References cited:
- EP-A1- 1 868 274
- EP-A1- 2 660 945
- EP-B1- 2 660 945
- US-A1- 2018 123 369
- US-A1- 2018 213 844
- US-A1- 2019 064 849
- US-A1- 2019 089 180

## Description

The present invention relates to an electrical system. In particular, the electrical system is used within an aerosol generating device.

Aerosol generating devices, such as electronic cigarettes, often include an electrical system with a battery for supplying power to a heating element. Within such systems, a known issue is that the battery may enter a state of deep discharge. For example, when a lithium-ion battery cell enters a state of deep discharge, internal degradations known as copper electrode dissolution may occur within the battery cell and short circuits may be created between cell electrodes. When such a battery is recharged, the cell is liable to overheating and thermal runaway, which can potentially pose a safety hazard.

There are many other battery conditions which carry potential safety risks and the operation of batteries displaying such conditions should generally be avoided.

US 2018/213844 A1 discloses aerosol delivery devices that may utilize electrically generated heat for the production of aerosol (e.g., smoking articles commonly referred to as electronic cigarettes).

An object of the present invention is to improve the safety of electrical systems including a battery within aerosol generating devices.

According to an aspect of the invention, there is provided an aerosol generating device comprising an electrical system, the electrical system comprising: a battery; and a control circuitry, wherein the control circuitry is configured to monitor a status of the battery during a discharge operation of the battery and set a flag if a fault in the battery is detected, the flag indicating that the battery is not in an operating condition, wherein the control circuitry is configured to check the flag when the electrical system is connected to an external power supply, wherein the control circuitry is configured to enable charging of the battery based on the flag, wherein the battery and the control circuitry are connectable to the external power supply via a first electrical path and a second electrical path respectively such that power can be independently supplied to the battery and the control circuitry, and wherein the electrical system is configured to supply power to the control circuitry from the external power supply via the second electrical path when the electrical system is connected to the external power supply, such that the flag can be checked without charging the battery .

In this way, it is possible to prevent a damaged or otherwise degraded battery from being charged, thereby improving the safety of an electrical system.

Existing strategies for responding to battery faults involve monitoring the charging curve of a battery to detect deep discharge or other hazardous battery conditions. However, such strategies will only detect a fault after charging of the cell has commenced. Thus, it is possible that power has already been supplied to a battery that has an internal short circuit or other fault. In the present invention, the control circuitry monitors the battery during discharge operation, for example when powering a heating element during a vaping operation of an aerosol generating device, and sets a flag within the control circuitry if a fault is detected. When the electrical system is subsequently connected to an external power supply with the intention of charging the aerosol generating device, the control circuitry checks the flag and only enables charging of the battery if the flag is present. As a result, charging of the battery is prevented from commencing if there is a fault in the battery, thereby ensuring that a battery in a hazardous condition does not receive any electrical power.

Moreover, the configuration of the electrical system is such that the control circuitry can be powered to check the flag without also supplying power to a possibly defective battery. In comparison, within known electrical systems, and in particular for aerosol generating devices, supplying power to the control circuitry also begins the charging process and it would not be possible to check the flag without also supplying power to a potentially hazardous battery.

Detecting a fault in the battery may comprise measuring the voltage of the battery with respect to time. A fault may be determined to have occurred when the voltage falls below a threshold voltage. In one example, for a lithium-ion battery, 3.0V may be a typical voltage at which the battery is considered to be discharged, 2.8V may be a typical threshold below which the battery is considered to have a fault, and 2.5V may be a typical voltage at which the battery has internal cell damage which cannot be recovered. However, the skilled person will appreciate that the threshold voltage will vary according to the type of battery and specific cell chemistry.

Alternatively, or additionally, detecting a fault in the battery may comprise monitoring the temperature of the battery. If the temperature of the battery exceeds a threshold temperature, the battery may be determined to have a fault. The skilled person will appreciate that the threshold temperature will vary according to the type of battery and the cell chemistry.

Preferably, the electrical system further comprises a battery charger circuitry, wherein the control circuitry is configured to send a signal to the battery charger circuitry based on the flag, the signal indicating that charging is enabled, and wherein the battery charger circuitry is configured to charge the battery when the signal indicating that charging is enabled is received from the control circuitry. In this way, the use of a battery charger circuitry ensures that power is efficiently and reliably supplied to the battery, whilst the signal receipt requirement ensures that power is not supplied to a damaged or degraded battery.

Preferably, charging the battery comprises supplying power to the battery along the first electrical path.

Preferably, the control circuitry is configured to modify the flag upon detecting that the battery has been replaced. In this way, a new battery that is not in a potentially dangerous operating condition is not prevented from being charged.

Preferably, the electrical system further comprises a voltage regulator for supplying power to the control circuitry. A voltage regulator has the ability to generate and maintain a constant current or voltage output.

In one example, the electrical system may be connectable to the external power supply by a USB connection. In particular, the voltage regulator and the battery charger circuitry may be connectable to the external power supply by the USB connection.

Preferably, the electrical system is configured to supply power to the control circuitry from the battery when the electrical system is not connected to the external power supply.

Preferably, the electrical system further comprises a heating element, and the control circuitry is configured to switch off power supply from the battery to the heating element when a fault is detected in the battery. In this way, continued operation of a damaged or otherwise degraded battery is avoided.

Preferably, the control circuitry is configured to switch off power supply from the battery to the heating element when the electrical system is connected to the external power supply.

Preferably, the electrical system further comprises a fuse, wherein the control circuitry is configured to activate the fuse when the fault detected in the battery is deemed to be non-recoverable, and wherein activating the fuse irreversibly disables charging of the battery.

Preferably, the control circuitry is further configured to activate the fuse when a threshold amount of time has elapsed since the flag was set and the fault in the battery is detected as still existing.

According to another aspect of the invention, there is provided a method of operating an aerosol generating device comprising an electrical system, the method comprising: monitoring, using a control circuitry, the status of a battery in the electrical system during a discharge operation of the battery; in response to detecting a fault in the battery, setting a flag indicating that the battery is not in an operating condition, wherein the battery and the control circuitry are connectable to an external power supply by a first electrical path and a second electrical path respectively such that power can be independently supplied to the control circuitry and the battery; in response to detecting that the electrical system has been connected to the external power supply, supplying power from the external power supply via the second electrical path to check the flag without charging the battery; and enabling charging of the battery based on the flag.

Preferably, the method further comprises sending a signal from the control circuitry to a battery charger circuitry indicating that charging is enabled; and in response to receiving the signal indicating that charging is enabled, charging the battery.

Preferably, the method further comprises clearing the flag upon detecting that the battery has been replaced.

Preferably, the method further comprises: supplying power to the control circuitry from the battery when the electrical system is not connected to the external power supply.

Preferably, the method further comprises: providing a heating element in the electrical system; switching off power supply to the heating element when a fault is detected in the battery; and/or switching off power supply from the battery to the heating element when the electrical system is connected to the external supply.

Preferably, the method further comprises: activating, using the control circuitry, a fuse in the electrical system when the fault detected in the battery is deemed to be non-recoverable, wherein activating the fuse irreversibly disables charging of the battery.

Preferably, the method further comprises: in response to detecting that a threshold amount of time has elapsed since the flag was set and that the fault in the battery still exists, activating, using the control circuitry, the fuse.

According to another aspect of the invention there is provided a non-transitory computer-readable memory medium comprising executable instructions which, when executed on a computer or processor in an aerosol generating device comprising an electrical system, cause the computer or processor to undertake steps comprising: monitoring, using a control circuitry, the status of a battery in the electrical system during a discharge operation of the battery; in response to detecting a fault in the battery, setting a flag indicating that the battery is not in an operating condition, wherein the battery and the control circuitry are connectable to an external power supply by a first electrical path and a second electrical path respectively such that power can be independently supplied to the control circuitry and the battery; in response to detecting that the electrical system has been connected to the external power supply, supplying power from the external power supply via the second electrical path to check the flag without charging the battery; and enabling charging of the battery based on the flag.

Embodiments of the invention are now described, by way of example, with reference to the drawings, in which:
Figure 1 is a block diagram of a prior art electrical system for an aerosol generating device;
Figure 2 is a block diagram of an electrical system for an aerosol generating device in an embodiment of the invention;
Figure 3A is a block diagram of the electrical system depicted in Figure 2, illustrating a first electrical path for supplying power from an external power supply to a battery and a second electrical path for supplying power from the external power supply to a control circuitry;
Figure 3B is a block diagram of the electrical system depicted in Figure 2, illustrating a third electrical path for supplying power from the battery to the control circuitry during a discharge operation of the battery;
Figure 4 is a flowchart showing method steps for operation of an electrical system for an aerosol generating device in an embodiment of the invention;
Figure 5 is a flowchart showing further method steps for operation of the electrical system; and
Figure 6 is a block diagram of an electrical system for an aerosol generating device in an embodiment of the invention.

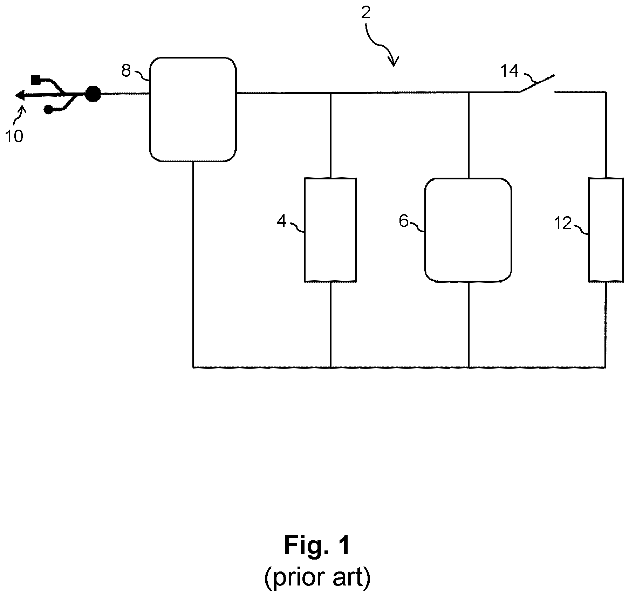

Figure 1 shows a prior art electrical system 2 for an aerosol generating device. The electrical system 2 comprises a battery 4, a control circuitry 6, a battery charging circuitry 8, a power connector 10, a heating element 12, and a switch 14.

In use, when the electrical system 2 is connected via the power connector 10 to an external power supply, the battery charging circuitry 8 delivers electrical power to wake up the control circuitry 6. However, as the battery 4 and the control circuitry 6 are connected in parallel and powered directly by the battery charging circuitry 8, the battery 4 also receives electrical power and begins charging. Hence, utilising any function of the control circuitry 6 also results in the battery 4 receiving electrical power.

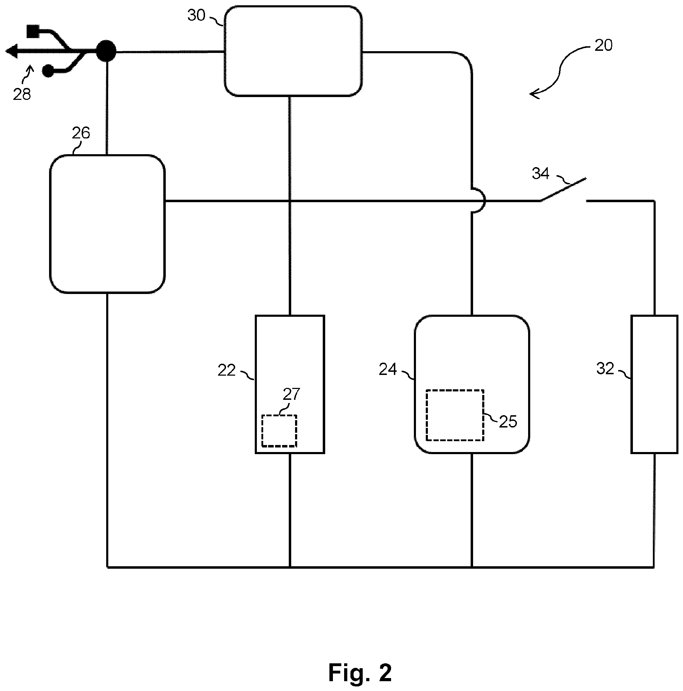

Figure 2 shows an electrical system 20 for an aerosol generating device in an embodiment of the invention. The electrical system comprises a battery 22, a control circuitry 24, a battery charging circuitry 26, a USB connector 28, a voltage regulator 30, a heating element 32 and a switch 34.

The USB connector 28 is connectable to an external power supply. The skilled person will appreciate that the USB connector 28 may be substituted for another suitable form of power connector, such as any AC power plug for connecting to primary alternating current (AC) power supply in a building, or any DC power plug for supplying direct current (DC) power.

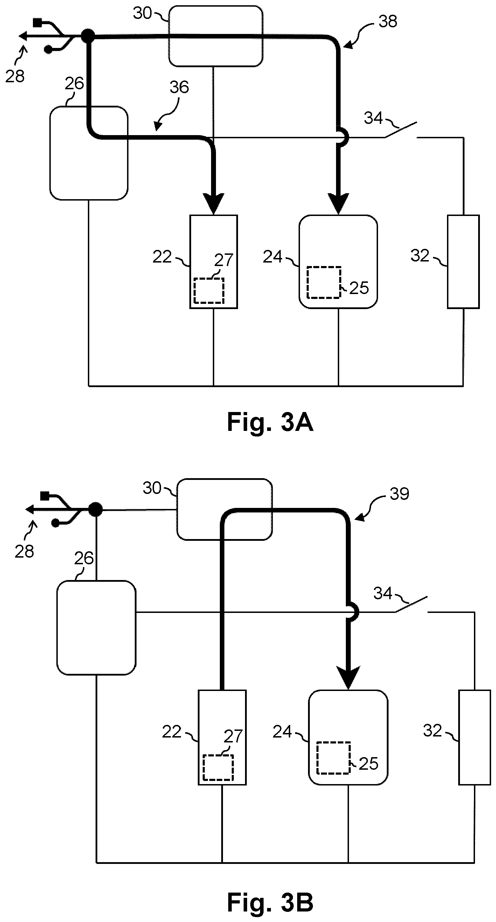

As illustrated in Figure 3A, electricity may be supplied from the USB connector 28 to the battery 22 along a first electrical path 36. The first electrical path 36 runs from the USB connector 28 to the battery 22 via the battery charging circuitry 26. Electricity may also be supplied from the USB connector 28 to the control circuitry 24 along a second electrical path 38. The second electrical path runs from the USB connector 28 to the control circuitry 24 via the voltage regulator 30. The first electrical path 36 and the second electrical path 38 are configured as separate, distinct electrical paths. Hence, when the USB connector 28 is connected to the external power supply, electricity may be supplied along the second electrical path 38 to power the control circuitry 24 without also supplying power to the battery 22. As used herein, the term 'electrical path' refers to a component suitable for transmitting electrical power by the conduction of electrons, such as a wire, cable or power line.

As illustrated in Figure 3B, a third electrical path 39 connects the battery 22 to the control circuitry 24 via the voltage regulator 30. The battery 22 may be a lithium-ion battery, a nickel cadmium battery, a nickel-metal hydride battery, a lead-acid battery, or any other type of rechargeable battery.

In use, the voltage regulator 30 either receives power from the external power supply (via the USB connector 28), or from the battery 22. The voltage regulator 30 can then supply electricity to the control circuitry 24 to wake up and power the control circuitry 24. The voltage regulator 30 is configured to power the control circuitry 24 with power from the USB connector 28 when the electrical system 20 is connected to the external power supply (i.e. power is supplied along the second electrical path 38), and configured to power the control circuitry 24 with power from the battery 22 otherwise (i.e. power is supplied along the third electrical path 39).

The voltage regulator 30 has the ability to generate and maintain a constant current or voltage output. It will be appreciated that, in alternative examples, the voltage regulator 30 may instead comprise a switch or other mechanism that allows the supply of electrical current to be controlled and/or regulated and directed along different electrical paths.

In this example, the control circuitry 24 is a microcontroller unit (MCU) and is used to control operation of the electrical system 20. The MCU includes one or more CPUs (processor cores) along with memory and programmable input/output peripherals. In other examples, the control circuitry 24 may comprise a separate microprocessor, memory, and input/output devices.

The control circuitry 24 is configured to monitor the status of the battery 22 during a discharge operation of the battery 22 and control one or more aspects of the electrical system based on the status of the battery 22. The term 'discharge operation' of the battery refers to the situation wherein the battery 22 is being used as power source to supply power to an electrical load or electrical component within the electrical system 20. Monitoring the status of the battery 22 may comprise monitoring one or more properties or characteristics of the battery 22, such as temperature, voltage or current, in order to detect a fault or abnormality within the battery 22.

For example, a fault may result from the battery 22 entering into a state of deep discharge leading to internal degradations of the battery, e.g. a short circuit. This may be detected by measuring the battery 22 voltage with respect to time, and determining when the voltage falls below a threshold voltage. The threshold voltage will vary according to the type of battery and specific cell chemistry. However, as an example, for a lithium-ion battery, 3.0V may be a typical voltage at which the battery is considered to be discharged, 2.8V may be a typical threshold below which the battery is considered to have a fault, and 2.5V may be a typical voltage at which the battery has internal cell damage which cannot be recovered. This internal damage is often referred to as copper (foil) dissolution.

A fault condition may also be determined by monitoring the temperature of the battery 22. A temperature sensor 27 may be used to measure the temperature of the battery. If the battery is operating abnormally, the temperature is likely to be high. Thus, if the temperature is detected to exceed a threshold temperature, the battery 22 may be determined to have a fault. Again, the threshold temperature will vary according to the type of battery and the cell chemistry.

A further example of detecting a fault may comprise detecting a battery capacity loss. Capacity loss (or capacity fading) is a phenomenon observed during rechargeable battery usage where the amount of charge a battery can deliver at the rated voltage decreases with use. For example, when the battery capacity fade exceeds approximately 60% - 70%, the battery may be considered to be too aged/damaged, and thus considered to have a fault.

In this case, the electrical system 20 is situated within an aerosol generating device, and the discharge operation refers to an aerosol generating operation (or vaping operation) wherein the battery 22 is providing power to the heating element 32. However, the skilled person will appreciate that the electrical system 20 may be used within alternative devices, and the heating element 32 may be substituted for other electrical components.

The control circuitry 24 is configured to set a flag in a data storage portion 25 of the control circuitry 23 when a fault is detected in the operating status of the battery 22. The data storage portion 25 may comprise volatile or non-volatile memory, or may comprise long-term storage. The flag provides an indication that a fault has been detected and that the battery 22 is not in an operating condition.

In this example, the flag is a form of status register set in an EEPROM (electrically erasable programmable read-only memory) of the MCU 24 and records the condition of a calculation performed by the MCU 24. Typically, a flag is defined as a 1 bit data in EEPROM; however, the number of bits may be increased to indicate the specific type of fault that has been detected.

The control circuitry 24 may also be configured to open the switch 34 when a fault is detected in the battery 22, thereby cutting off the supply of electricity to the heating element 32 and improving the safety of the aerosol generating device.

In one example, the electrical system 20 may further comprise a data line connecting the control circuitry 24 to the electrical system 20 which is configured to provide voltage information to the control circuitry 24.

In order to charge the battery 22, the electrical system 20 of the aerosol generating device may be connected to an external power supply by the USB connector 28. The voltage regulator 30 receives power from the USB connector 28 and generates a CC (constant current) output which is used to wake up the control circuitry 24 by the supply of electricity along the second electrical path 38. As the battery 22 is connected to the USB connector 28 by the first electrical path 36, which is separate to the second electrical path 38, the control circuitry 24 can be powered without also charging the battery 22.

In response to being powered up by the voltage regulator 30 when connected to an external power supply, the control circuitry 22 is configured to check the flag. If the flag is present, the control circuitry 24 will not enable charging of the battery 22. If the flag is cleared or not present, the control circuitry 24 will enable charging of the battery 22.

Enabling charging of the battery 22 comprises sending a signal to the battery charging circuitry 26, wherein the signal indicates that charging of the battery 22 is enabled. The battery charging circuitry 26 is configured to only charge the battery 22 when the charging enabled signal has been received from the control circuitry 24. Charging of the battery 22 comprises supplying power along the first electrical path 36 to the battery 22. The battery charging circuitry 26 will not charge the battery 22 if a signal has not been received. Hence, the configuration ensures that the charging process cannot begin if the battery 22 has a detected fault, thereby improving the safety of the aerosol generating device. This method of operation is facilitated by the separate electrical paths 36, 38 for the battery 22 and control circuitry 24 respectively which allow the control circuitry 24 to be powered to check the flag without also charging the battery 22.

In this example, the battery charging circuitry 26 is a battery charger IC (integrated circuit).

For other general purposes, the control circuitry 24 may be configured to switch off power supply from the battery 22 to the heating element 32 when the electrical system 20 is connected to an external power source through the USB connector 28. This may be achieved by opening the switch 34. Moreover, the control circuitry 24 may be configured to clear the flag if the control circuitry 24 detects that the battery 22 has been replaced.

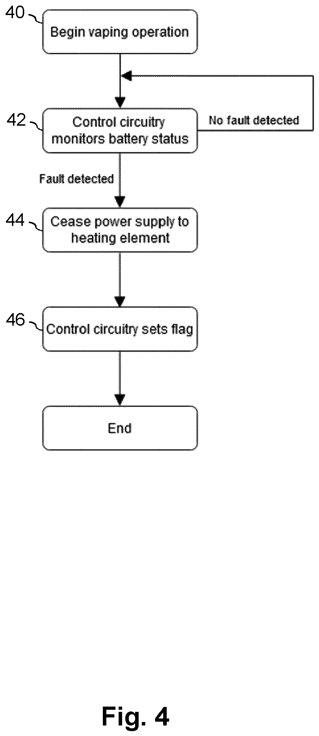

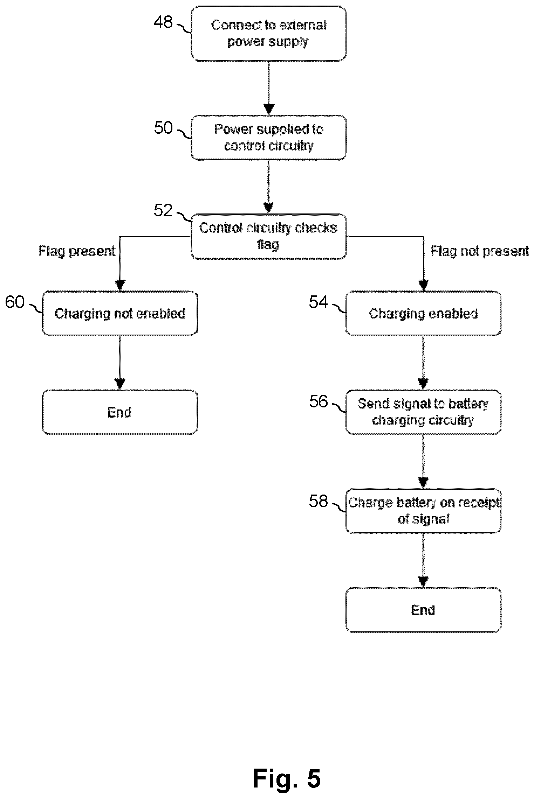

Figures 4 and 5 illustrate a method of operation for an electrical system 20 of an aerosol generating device in an embodiment of the invention.

Referring to Figure 4, the method commences at step 40 when the aerosol generating device enters into a vaping or aerosol generating mode of operation. During the vaping mode of operation, the battery 22 is used as a power source to supply electrical power to the heating element 32. The battery also supplies electricity along the third electrical path 39 to power the control circuitry 24.

At step 42, the control circuitry 24 monitors the status of the battery 22. For example, the control circuitry 24 may monitor the battery 22 voltage over time in order to detect a deep discharge state. If no fault is detected, the monitoring and vaping operation continues.

If a fault is detected, the switch 34 is opened and the supply of power from the battery 22 to the heating element 32 is stopped so that the aerosol generating device ends its vaping operation. In addition, at step 46, the control circuitry 24 sets a flag in the control circuitry 24 which indicates that the battery 22 is not in an operating condition.

Referring to Figure 5, the method continues at step 48 when the aerosol generating device is connected to an external power supply by the USB connector 28, with the intention of charging the battery 22 within the aerosol generating device.

Upon being connected to an external power supply, at step 50, a CC output is generated by the voltage regulator 30 and supplied to the control circuitry 24 along the second electrical path 38. As the first electrical path 36 and the second electrical path 38 comprises separate conductions paths, the control circuitry 24 can be woken and powered without supplying power to the battery 22.

At step 52, when the control circuitry 24 has been woken, the control circuitry 24 checks the flag.

If the flag is cleared or not present, the method continues at step 54 and charging of the battery 22 is enabled. At step 56, the control circuitry 24 sends a signal to the battery charging circuitry 26 indicating that charging of the battery 22 is enabled. At step 58, when the battery charging circuitry 26 receives the signal indicating that charging is enabled, the battery charging circuitry 26 proceeds to charge the battery 22 by supplying the power along the first electrical path 36.

Alternatively, if the flag is not cleared at step 52, the method continues at step 60 and charging of the battery is not enabled 60.

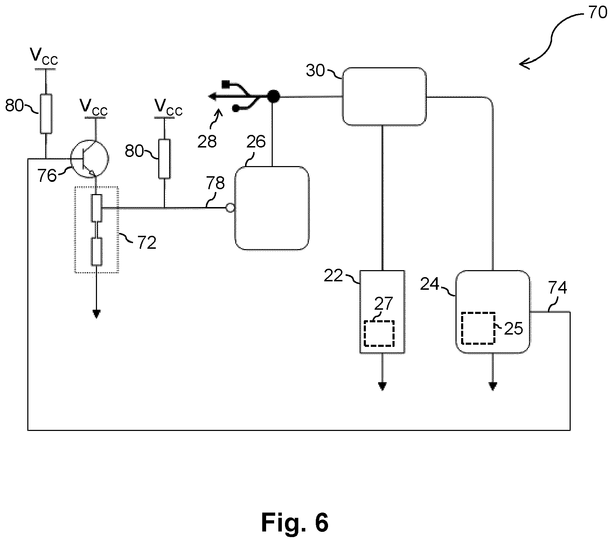

Figure 6 shows an electrical system 70 according to another embodiment of the invention. The electrical system 70 comprises corresponding features to those described with reference to Figures 2 to 5, and is configured to operate substantially in line with the method of Figures 4 and 5 under certain conditions as described below. For ease of reference, however, several of the previously described connections and features have been omitted from Figure 6. Nonetheless, the skilled person will appreciate that the omitted features, such as the heating element 32, may be used in conjunction with the additional features of this embodiment.

The electrical system 70 differs from the previous embodiment in that the electrical system 70 further comprises a fuse 72 for disabling charging of the battery 22. The fuse 72 is present in addition to the previously described flag that may be set in the control circuitry 24 for disabling charging of the battery 22. That is, the electrical system 70 utilises both hardware and software means for disabling charging of the battery 22 when a fault is detected in the battery 22.

In particular, the previously described flag mechanism provides a first level of protection for preventing charging of the battery 22 when a fault is detected in the battery 22, wherein the fault is caused by a battery condition that is deemed as being (potentially) recoverable. The fuse 72 provides a second level of protection for preventing charging of the battery 22 when a fault is detected in the battery 22, wherein the fault is caused by a battery condition that is deemed as not being recoverable.

Example damages to the battery 22 that may be deemed as non-recoverable include internal short circuits. For example, as previously discussed, short circuits may result from the battery 22 entering into a state of deep discharge leading to internal degradations of the battery 22. This may be detected by measuring the battery 22 voltage with respect to time, and determining when the voltage falls below a threshold voltage. Another indication of permanent, non-recoverable damage is the detection of voltage drops during the charging process. Such voltage drops indicate that the battery 22 has internal short-circuits.

On the other hand, an example of damage to the battery 22 that may be deemed as recoverable are capacity losses due to lithium plating. Lithium plating occurs under strenuous or sub-optimal charging conditions. Such losses of capacity may be recovered by preventing operation of the battery 22 for a period of time, e.g. several days, or performing one or more charging cycles and monitoring the capacity evolution over time. However, under some circumstances, capacity losses due to lithium plating may not be recoverable, for example if the internal damages are too severe.

In this embodiment of the invention, if a non-recoverable fault condition of the battery 22 is detected, such as the defection of voltage drops during charging or the detection that the battery 22 has entered a state of deep-discharge, the fuse 72 is activated by the control circuitry 24 such that charging of the battery 22 is permanently disabled.

Otherwise, if a fault condition of the battery 22 is detected that is not deemed to be non-recoverable, e.g. a capacity loss of the battery 22 is detected wherein the capacity loss is above a threshold amount, the electrical system 70 operates according to the previously described embodiment. That is, a flag is set indicating that the battery 22 is not in an operating condition, thereby preventing charging of the battery 22 whilst the flag is present.

However, after a period of time has elapsed, if the fault condition is detected to still exist the damage to the battery 22 may be deemed as being non-recoverable. In this case, the fuse 72 is activated by the control circuitry 24 such that charging of the battery 22 is permanently disabled. For example, the fuse 72 may be activated if, after a threshold amount of time has elapsed, the capacity of the battery 22 remains below a threshold capacity, e.g. less than 50 to 40% of nominal capacity. The control circuitry 24 may comprise a timer configured to monitor the elapsed time or, alternatively or additionally, the control circuitry 24 may estimate the elapsed time by monitoring the voltage evolution of the battery 22.

As will be appreciated by the skilled person, the fuse 72 is a physical component that is configured to break if the current exceeds a predetermined level. For example, the fuse 72 may consist of a strip of wire that is configured to melt above the predetermined level of current. In particular, the fuse 72 may comprise a copper track with a narrower central portion, as illustrated in Figure 6.

In the specific implementation of the electrical circuit 70 illustrated in Figure 6, the electrical system 70 comprises an I/O line 74, a transistor 76 (e.g. an NPN transistor), an enable line 78, resistors 80, and positive supply voltages V_{cc}. The I/O line 74 extends from the control circuitry 24 to the transistor 74. A first positive supply voltage V_{cc} is connected to the I/O line 74 via a first resistor 80. The transistor 76 is connected to the fuse 72 and a second positive supply voltage V_{cc}. The fuse 72 is connected to the charging circuitry 26 via the enable line 78. A third positive supply voltage V_{CC} is connected to the enable line 78 via a second resistor 80.

When a fault is detected in the battery 22 that is deemed as being non-recoverable, or a threshold amount of time has elapsed since a flag has been set, the control circuitry 24 is configured to send a signal along the I/O line 74 to turn the transistor "ON" such that a maximum current flows through the fuse 72. In this way, the fuse 72 is blown (i.e. activated) and the enable line 78, which is connected to the transistor 76 via a portion of the fuse 72, provides a control signal (e.g. set to high) to the charging circuitry 26 which permanently disables charging of the battery 22.

Of course, the skilled person will appreciate the specific configuration of the electrical system 70 including the fuse 72 illustrated in Figure 6 is an exemplary configuration, and various modifications may be made to the electrical system 70.

## Claims

1. An aerosol generating device comprising an electrical system (20), the electrical system (20) comprising:
a battery (22); and
a control circuitry (24), wherein the control circuitry (24) is configured to monitor a status of the battery (22) during a discharge operation of the battery (22) and set a flag if a fault in the battery (22) is detected, the flag indicating that the battery (22) is not in an operating condition,
wherein the control circuitry (24) is configured to check the flag when the electrical system (20) is connected to an external power supply, and
wherein the control circuitry (24) is configured to enable charging of the battery (22) based on the flag,
**characterised in that**:
the battery (22) and the control circuitry (24) are connectable to the external power supply via a first electrical path (36) and a second electrical path (38) respectively such that power can be independently supplied to the battery (22) and the control circuitry (24), and
the electrical system (20) is configured to supply power to the control circuitry (24) from the external power supply via the second electrical path (38) when the electrical system (20) is connected to the external power supply, such that the flag can be checked without charging the battery (22).

2. The aerosol generating device of claim 1, further comprising a battery charger circuitry (26), wherein the control circuitry (24) is configured to send a signal to the battery charger circuitry (26) based on the flag, the signal indicating that charging is enabled, and
wherein the battery charger circuitry (26) is configured to charge the battery (22) when the signal indicating that charging is enabled is received from the control circuitry (24).

3. The aerosol generating device of claim 2, wherein charging the battery (22) comprises supplying power to the battery (22) along the first electrical path (36).

4. The aerosol generating device of any preceding claim, wherein the control circuitry (24) is configured to modify the flag upon detecting that the battery (22) has been replaced.

5. The aerosol generating device of any of claims 1 to 4, wherein the electrical system (20) is configured to supply power to the control circuitry (24) from the battery (22) when the electrical system (20) is not connected to the external power supply.

6. The aerosol generating device of any preceding claim, wherein the electrical system (20) further comprises a heating element (32), and wherein the control circuitry (24) is configured to switch off power supply from the battery (22) to the heating element (32) when a fault is detected in the battery (22).

7. The aerosol generating device of claim 6, wherein the control circuitry (24) is configured to switch off power supply from the battery (22) to the heating element (32) when the electrical system (20) is connected to the external power supply.

8. The aerosol generating device of any preceding claim, wherein the electrical system (70) further comprises a fuse (72), wherein the control circuitry (24) is configured to activate the fuse (72) when the fault detected in the battery (22) is deemed to be non-recoverable, and wherein activating the fuse (72) irreversibly disables charging of the battery (22).

9. The aerosol generating device of claim 8, wherein the control circuitry (24) is further configured to activate the fuse (72) when a threshold amount of time has elapsed since the flag was set and the fault in the battery (22) is detected as still existing.

10. A method of operating an aerosol generating device comprising an electrical system, the method comprising:
monitoring (42), using a control circuitry, the status of a battery in the electrical system during a discharge operation of the battery;
in response to detecting a fault in the battery, setting (46) a flag indicating that the battery is not in an operating condition,
wherein the battery and the control circuitry are connectable to an external power supply by a first electrical path and a second electrical path respectively such that power can be independently supplied to the control circuitry and the battery;
in response to detecting that the electrical system has been connected to the external power supply, supplying (50) power to the control circuitry from the external power supply via the second electrical path to check the flag without charging the battery; and
enabling (54) charging of the battery based on the flag.

11. The method of claim 10, further comprising:
sending (56) a signal from the control circuitry to a battery charger circuitry indicating that charging is enabled; and
in response to receiving the signal indicating that charging is enabled, charging (58) the battery.

12. The method of claim 10 or claim 11, further comprising clearing the flag upon detecting that the battery has been replaced.

13. The method of any of claims 10 to 12, further comprising:
supplying power to the control circuitry from the battery when the electrical system is not connected to the external power supply.

14. The method of any of claims 10 to 13, further comprising:
activating, using the control circuitry, a fuse in the electrical system when the fault in the battery is deemed to be non-recoverable, wherein activating the fuse irreversibly disables charging of the battery.

15. A non-transitory computer-readable memory medium comprising executable instructions which, when executed on a computer or processor in an aerosol generating device comprising an electrical system, cause the computer or processor to undertake steps comprising:
monitoring, using a control circuitry, the status of a battery in the electrical system during a discharge operation of the battery;
in response to detecting a fault in the battery, setting a flag indicating that the battery is not in an operating condition,
wherein the battery and the control circuitry are connectable to an external power supply by a first electrical path and a second electrical path respectively such that power can be independently supplied to the control circuitry and the battery;
in response to detecting that the electrical system has been connected to the external power supply, supplying power to the control circuitry from the external power supply via the second electrical path to check the flag without charging the battery; and
enabling charging of the battery based on the flag.

## Patentansprüche

1. Aerosolerzeugungsvorrichtung, die ein elektrisches System (20) umfasst, wobei das elektrische System (20) umfasst:
Eine Batterie (22); und
eine Steuerschaltung (24), wobei die Steuerschaltung (24) ausgelegt ist, einen Status der Batterie (22), während eines Entladevorgangs der Batterie (22) zu überwachen und ein Flag zu setzen, wenn ein Fehler in der Batterie (22) erkannt wird, wobei das Flag darauf hindeutet, dass die Batterie (22) nicht in einem Betriebszustand ist,
wobei die Steuerschaltung (24) ausgelegt ist, das Flag zu überprüfen, wenn das elektrische System (20) mit einer externen Stromversorgung verbunden wird, und
wobei die Steuerschaltung (24) ausgelegt ist, Laden der Batterie (22) auf dem Flag basierend zu ermöglichen,
**dadurch gekennzeichnet, dass**:
Die Batterie (22) und die Steuerschaltung (24) an die externe Stromversorgung über einen ersten elektrischen Pfad (36) bzw. einen zweiten elektrischen Pfad (38) so anschließbar sind, dass der Strom unabhängig zur Batterie (22) und der Steuerschaltung (24) geliefert werden kann, und
das elektrische System (20) ausgelegt ist, Strom an die Steuerschaltung (24) ab der externen Stromversorgung über den zweiten elektrischen Strompfad (38) zu liefern, wenn das elektrische System (20) an die externe Stromversorgung angeschlossen ist, sodass sich das Flag überprüfen lässt ohne die Batterie (22) zu laden.

2. Aerosolerzeugungsvorrichtung nach Anspruch 1, die ferner eine Batterieladeschaltung (26) umfasst, wobei die Steuerschaltung (24) ausgelegt ist, auf dem Flag basierend, ein Signal an die Batterieladeschaltung (26) zu senden, wobei das Signal andeutet, dass der Ladevorgang aktiviert ist, und
wobei die Batterieladeschaltung (26) ausgelegt ist, die Batterie (22) zu laden, wenn das Signal, welches andeutet, dass der Ladevorgang aktiviert ist, von der Steuerschaltung (24) empfangen wird.

3. Aerosolerzeugungsvorrichtung nach Anspruch 2, wobei das Laden der Batterie (22) die Stromzufuhr zur Batterie (22) entlang des ersten elektrischen Pfads (36) umfasst.

4. Aerosolerzeugungsvorrichtung nach irgendeinem vorhergehenden Anspruch, wobei die Steuerschaltung (24) ausgelegt ist, nach Erkennung, dass die Batterie (22) ersetzt worden ist, das Flag zu modifizieren.

5. Aerosolerzeugungsvorrichtung nach irgendeinem der Ansprüche 1 bis 4, wobei das elektrische System (20) ausgelegt ist, Strom aus der Batterie (22) zur Steuerschaltung (24) zu liefern, wenn das elektrische (20) nicht an die externe Stromversorgung angeschlossen ist.

6. Aerosolerzeugungsvorrichtung nach irgendeinem vorhergehenden Anspruch, wobei das elektrische System (20) ferner ein Heizelement (32) umfasst, und wobei die Steuerschaltung (24) ausgelegt ist, die Stromversorgung von der Batterie (22) zum Heizelement (32) abzuschalten, wenn ein Fehler in der Batterie (22) erkannt wird.

7. Aerosolerzeugungsvorrichtung nach Anspruch 6, wobei die Steuerschaltung (24) ausgelegt ist, die Stromversorgung von der Batterie (22) zum Heizelement (32) abzuschalten, wenn das elektrische System (20) an die externe Stromversorgung angeschlossen ist.

8. Aerosolerzeugungsvorrichtung nach irgendeinem vorhergehenden Anspruch, wobei das elektrische System (70), welches ferner eine Sicherung (72) umfasst, wobei die Steuerschaltung (24) ausgelegt ist, die Sicherung (72) zu aktivieren, wenn der in der Batterie (22) erkannte Fehler als nicht behebbar erachtet wird, und wobei die Aktivierung der Sicherung (72) das Laden der Batterie (22) irreversibel außerstand setzt.

9. Aerosolerzeugungsvorrichtung nach Anspruch 8, wobei die Steuerschaltung (24) ferner ausgelegt ist, die Sicherung (72) zu aktivieren, wenn seit dem Setzen des Flags eine bestimmte Zeitspanne verstrichen ist und der Fehler in der Batterie (22) als weiterhin vorhanden erkannt wird.

10. Verfahren zum Betrieb einer aerosolerzeugenden Vorrichtung, die ein elektrisches System umfasst, wobei das Verfahren umfasst:
Überwachen (42), unter Verwendung einer Steuerschaltung, des Status einer Batterie im elektrischen System, während eines Entladevorgangs der Batterie;
als Reaktion auf das Erkennen eines Fehlers in der Batterie, Setzen (46) eines Flags, das andeutet, dass sich die Batterie nicht in einem Betriebszustand befindet,
wobei die Batterie und die Steuerschaltung an eine externe Stromversorgung durch einen ersten elektrischen Pfad bzw. einen zweiten elektrischen Pfad so anschließbar sind, dass der Strom unabhängig zur Steuerschaltung und der Batterie geliefert werden kann;
als Reaktion auf das Erkennen, dass das elektrische System an die externe Stromversorgung angeschlossen worden ist, Zuführen (50) von Strom zur Steuerschaltung ab der externen Stromversorgung über den zweiten elektrischen Pfad, um das Flag zu prüfen, ohne die Batterie zu laden; und
Ermöglichen (54) des Ladens der Batterie auf Basis des Flags.

11. Verfahren nach Anspruch 10, ferner umfassend:
Senden (56) eines Signals von der Steuerschaltung zu einer Batterieladeschaltung, das anzeigt, dass der Ladevorgang aktiviert ist; und
als Reaktion auf den Empfang eines Signals, das anzeigt, das der Ladevorgang aktiviert ist, Laden (58) der Batterie.

12. Verfahren nach Anspruch 10 oder Anspruch 11, das ferner das Löschen des Flags umfasst, wenn erkannt wird, dass die Batterie ausgetauscht worden ist.

13. Das Verfahren nach einem der Ansprüche 10 bis 12, ferner umfassend:
Zuführen von Strom aus der Batterie zur Steuerschaltung, wenn das elektrische System nicht an die externe Stromversorgung angeschlossen ist.

14. Das Verfahren nach einem der Ansprüche 10 bis 13, ferner umfassend:
Aktivieren, unter Verwendung der Steuerschaltung, einer Sicherung im elektrischen System, wenn der Fehler in der Batterie als nicht behebbar erachtet wird, wobei die Aktivierung der Sicherheit das Laden der Batterie irreversibel außerstand setzt.

15. Nichtflüchtiges computerlesbares Speichermedium, welches ausführbare Anweisungen umfasst, die, wenn auf einem Computer oder Prozessor in einer aerosolerzeugenden Vorrichtung ausgeführt, die ein elektrisches System umfasst, bewirken, dass der Computer oder Prozessor Schritte unternimmt, die umfassen:
Überwachen, unter Verwendung einer Steuerschaltung, des Status einer Batterie im elektrischen System, während eines Entladevorgangs der Batterie;
als Reaktion auf das Erkennen eines Fehlers in der Batterie, Setzen eines Flags, das andeutet, dass sich die Batterie nicht in einem Betriebszustand befindet,
wobei die Batterie und die Steuerschaltung an eine externe Stromversorgung durch einen ersten elektrischen Pfad bzw. einen zweiten elektrischen Pfad so anschließbar sind, dass der Strom unabhängig zur Steuerschaltung und der Batterie geliefert werden kann;
als Reaktion auf das Erkennen, dass das elektrische System an die externe Stromversorgung angeschlossen worden ist, Zuführen von Strom zur Steuerschaltung ab der externen Stromversorgung über den zweiten elektrischen Pfad, um das Flag zu überprüfen, ohne die Batterie zu laden; und
Ermöglichen des Ladens der Batterie auf Basis des Flags.

## Revendications

1. Un dispositif de génération d'un aérosol qui se compose d'un système électrique (20), et ce système électrique (20) comporte les éléments suivants :
une batterie (22) et
des circuits de commande (24), et ces circuits de commande (24) sont configurés pour surveiller le statut de la batterie (22) lors d'une opération de décharge de la batterie (22) et affichent un avertissement lorsqu'une anomalie de la batterie (22) est détectée, et cet avertissement indique que la batterie (22) n'est pas en état de marche,
et ces circuits de commande (24) sont configurés pour vérifier la présence éventuelle d'un avertissement lorsque le système électrique (20) est raccordé à une source extérieure d'alimentation en courant électrique, et
les circuits de commande (24) sont configurés pour permettre la recharge de la batterie (22), en fonction de tout avertissement présent,
**se caractérisant par le fait que** :
la batterie (22) et les circuits de commande (24) peuvent se brancher sur la source extérieure d'alimentation en courant électrique par l'entremise d'un premier circuit électrique (36) et d'un deuxième circuit électrique (38) afin d'alimenter respectivement en courant électrique, de manière indépendante, la batterie (22) et les circuits de commande (24), et
le système électrique (20) est configuré pour alimenter en courant électrique les circuits de commande (24) par le biais d'une source extérieure d'alimentation en courant électrique, en faisant appel au deuxième circuit électrique (38) lorsque le système électrique (20) est raccordé à la source extérieure d'alimentation en courant électrique et il est alors possible de vérifier la présence éventuelle d'un avertissement sans recharger la batterie (22).

2. Le dispositif de génération d'un aérosol que décrit la revendication 1, si ce n'est qu'il comporte, en outre, des circuits pour chargeur de batterie (26), que les circuits de commande (24) sont configurés pour envoyer un signal aux circuits pour chargeur de batterie (26), en fonction de l'avertissement éventuel qui s'affiche et ce signal indique l'activation d'une recharge, et
les circuits du chargeur de batterie (26) sont configurés pour recharger la batterie (22) lorsque le signal indiquant qu'une recharge a été activée est reçu, en provenance des circuits de commande (24).

3. Le dispositif de génération d'un aérosol que décrit la revendication 2, si ce n'est que la recharge de la batterie (22) passe par la fourniture d'un courant électrique à la batterie (22) le long du premier circuit électrique (36).

4. Le dispositif de génération d'un aérosol que décrit l'une ou l'autre des revendications précédentes, si ce n'est que les circuits de commande (24) sont configurés pour modifier l'avertissement dès que le remplacement de la batterie (22) a été détecté.

5. Le dispositif de génération d'un aérosol que décrit l'une ou l'autre des revendications 1 à 4, si ce n'est que le système électrique (20) est configuré pour fournir un courant électrique aux circuits de commande (24) depuis la batterie (22) lorsque le système électrique (20) n'est pas raccordé à la source extérieure d'alimentation en courant.

6. Le dispositif de génération d'un aérosol que décrit l'une ou l'autre des revendications précédentes, si ce n'est que le système électrique (20) comporte, en outre, un élément chauffant (32) et que les circuits de commande (24) sont configurés pour couper le courant électrique que fournit la batterie (22) à l'élément chauffant (32) lorsqu'une anomalie est détectée dans la batterie (22).

7. Le dispositif de génération d'un aérosol que décrit la revendications 6, si ce n'est que les circuits de commande (24) sont configurés pour couper le courant électrique que fournit la batterie (22) à l'élément chauffant (32) lorsque le système électrique (20) est raccordé à la source extérieure d'alimentation en courant.

8. Le dispositif de génération d'un aérosol que décrit l'une ou l'autre des revendications précédentes, si ce n'est que le système électrique (70) comporte, en outre, un fusible (72), que les circuits de commande(24) sont configurés pour activer le fusible (72) lorsqu'on considère qu'il n'est pas possible de rectifier l'anomalie détectée dans la batterie (22) et que l'activation du fusible (72) entraîne une désactivation irréversible de la recharge de la batterie (22).

9. Le dispositif de génération d'un aérosol que décrit la revendication 8, si ce n'est que les circuits de commande (24) sont, en outre, configurés pour activer le fusible (72) lorsqu'un certain seuil de temps s'est écoulé depuis l'affichage d'un avertissement et lorsque l'anomalie détectée dans la batterie (22) reste présente.

10. Un procédé d'utilisation d'un dispositif de génération d'un aérosol comportant un système électrique, et ce procédé se compose des éléments suivants :
la surveillance (42), à l'aide de circuits de commande, du statut d'une batterie dans le système électrique lors d'une opération de décharge de la batterie
en réponse à la détection d'une anomalie dans la batterie, la programmation (46) d'un avertissement indiquant que la batterie n'est pas en état de marche,
et la batterie et les circuits de commande peuvent se brancher sur une source extérieure d'alimentation en courant électrique par l'entremise d'un premier circuit électrique et d'un deuxième circuit électrique afin d'alimenter respectivement en courant électrique, de manière indépendante, les circuits de commande et la batterie
en réponse à la détection du fait que le système électrique est raccordé à la source extérieure d'alimentation en courant électrique, la fourniture (50) d'un courant électrique aux circuits de commande depuis cette source d'alimentation en courant électrique, par l'entremise du deuxième circuit électrique, pour vérifier l'avertissement présent, sans recharger la batterie, et
l'activation (54) de la recharge de la batterie, en fonction de l'avertissement présent.

11. Le procédé que décrit la revendication 10, si ce n'est qu'il comporte en outre :
l'envoi (56) d'un signal en provenance des circuits de commande et à destination des circuits d'un chargeur de batterie, pour indiquer qu'une recharge est activée, et
en réponse à la réception du signal indiquant que la recharge est activée, la recharge (58) de la batterie.

12. Le procédé que décrit la revendication 10 ou 11, si ce n'est qu'il comporte, en outre, la suppression de l'avertissement dès la détection que la batterie a été remplacée.

13. Le procédé que décrit l'une ou l'autre des revendications 10 à 12, si ce n'est qu'il comporte, en outre :
la fourniture d'un courant aux circuits de commande, en provenance de la batterie, lorsque le système électrique n'est pas raccordé à la source extérieure d'alimentation en courant.

14. Le procédé que décrit l'une ou l'autre des revendications 10 à 13, si ce n'est qu'il comporte, en outre :
L'activation, à l'aide des circuits de commande, d'un fusible dans le système électrique lorsque l'anomalie dans la batterie est considérée comme ne pouvant pas être corrigée, alors que cette activation du fusible entraîne une désactivation irréversible de la batterie.

15. Un support de mémoire électronique non transitoire qui contient des instructions exécutables qui, lorsqu'elles sont exécutées par un ordinateur ou un processeur implanté dans un dispositif de génération d'un aérosol comportant un système électrique, entraîne la réalisation des opérations suivantes par cet ordinateur ou ce processeur :
la surveillance, à l'aide de circuits de commande, du statut d'une batterie dans le système électrique lors d'une opération de décharge de la batterie
en réponse à la détection d'une anomalie dans la batterie, la programmation d'un avertissement indiquant que la batterie n'est pas en état de marche,
et la batterie et les circuits de commande peuvent se brancher sur une source extérieure d'alimentation en courant électrique par l'entremise d'un premier circuit électrique et d'un deuxième circuit électrique afin d'alimenter respectivement en courant électrique, de manière indépendante, les circuits de commande et la batterie
en réponse à la détection du fait que le système électrique est raccordé à la source extérieure d'alimentation en courant électrique, la fourniture d'un courant électrique aux circuits de commande depuis cette source d'alimentation en courant électrique, par l'entremise du deuxième circuit électrique, pour vérifier l'avertissement présent, sans recharger la batterie, et
l'activation de la recharge de la batterie, en fonction de l'avertissement présent.
